(19) Europäisches Patentamt
European Patent Office
Office européen des brevets

(11) Veröffentlichungsnummer: **0 276 803 B1**

(12) **EUROPÄISCHE PATENTSCHRIFT**

(45) Veröffentlichungstag der Patentschrift: **16.10.91**

(51) Int. Cl.⁵: **C07D 243/08**, A61K 31/55

(21) Anmeldenummer: **88101054.0**

(22) Anmeldetag: **26.01.88**

# (54) 1-(4-Hydroxy-3,5-di-tert.-butyl-benzoyl)-homopiperazin, verschiedene von dessen Derivaten, Verfahren zur Herstellung dieser Verbindungen, die sie enthaltenden Arzneimittel und ihre Verwendung.

(30) Priorität: **30.01.87 DE 3702755**

(43) Veröffentlichungstag der Anmeldung:
**03.08.88 Patentblatt 88/31**

(45) Bekanntmachung des Hinweises auf die Patenterteilung:
**16.10.91 Patentblatt 91/42**

(84) Benannte Vertragsstaaten:
**AT BE CH DE ES FR GB GR IT LI LU NL SE**

(56) Entgegenhaltungen:
**US-A- 4 066 614**
**US-A- 4 404 302**

**CHEMICAL ABSTRACTS, Band 77, Nr. 7, 14. August 1972, Columbus, Ohio, USA; T. TAKAHASHI et al.: "Homopiperazine compunds", Seite 498, Spalte 1, Zusammenfassung-Nr. 48520m**

**CHEMICAL ABSTRACTS, Band 75, Nr. 5, 2. August 1971, Columbus, Ohio, USA; T. TAKAHASHI et al.: "1,4-Disubstituted homopiperazines", Seite 526, Spalte 1, Zusammenfassung-Nr. 36162s**

(73) Patentinhaber: **HOECHST AKTIENGESELLSCHAFT**
**Postfach 80 03 20**
**W-6230 Frankfurt am Main 80(DE)**

(72) Erfinder: **Wolf, Erhard, Dr.**
**Ostpreussenstrasse 17**
**W-6238 Hofheim am Taunus(DE)**
Erfinder: **Rossmanith, Erhard**
**Spechtstrasse 4**
**W-6231 Schwalbach/Taunus(DE)**
Erfinder: **Bartlett, Robert R., Dr.**
**Sandbergstrasse 20**
**W-6100 Darmstadt(DE)**
Erfinder: **Schleyerbach, Rudolf, Dr.**
**Finkenweg 10**
**W-6238 Hofheim am Taunus(DE)**

**EP 0 276 803 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die vorliegende Erfindung betrifft 1-(4-Hydroxy-3,5-di-tert.-butylbenzoyl)-homopiperazin, verschiedene von dessen Derivaten, Verfahren zur Herstellung dieser Verbindungen und ihre Verwendung als Wirkstoffe in Arzneimitteln hauptsächlich zur Behandlung von Erkrankungen des rheumatischen Formenkreises, insbesondere der schweren Verlaufsformen wie der bisher kaum therapierbaren chronischen Graft-versus-Host-Krankheiten und von Immunkomplex-mediierten Autoimmunerkrankungen wie zum Beispiel der systemische Lupus erythematodes, der Immunkomplexglomerulonephritis und der Typ-I-Diabetes.

Zur Behandlung von Erkrankungen des rheumatischen Formenkreises werden vorzugsweise nichtsteroidale Antirheumatika ( = NSAID) eingesetzt, welche sich bevorzugt aus den Stoffklassen der Aralkylcarbonsäuren, Anthranilsäure, Oxicame, Salicylsäure und Pyrazole rekrutieren. Diese Antirheumatika vermögen zwar den mit der Krankheit verbundenen Schmerz, die Entzündung und Schwellung günstig zu beeinflussen, sie können jedoch zu gravierenden Nebenwirkungen führen (vgl.K. Brune, Eur. J. Rheumatol, Inflam. 5 (1982), S 335 - 349; Litera Rheumatologica 3, Symposiumsbericht, F.J. Wagenhäuser, Nutzen und Risiken der Antirheumatika, Basel, 1985): Außer Nierenfunktionsstörungen und allergischen Reaktionen, wie Asthmaanfälle, werden vor allem gastrointestinale Störungen beobachtet, wobei die chemische Struktur der verwendeten NSAID von untergeordneter Bedeutung zu sein scheint(Coles, L.S. et al. Amer. J Med. 74, 1983, S. 820) Nachteilig ist ferner die oft geringe therapeutische Breite dieser Verbindungen.

Darüber hinaus sind die schweren Verlaufsformen mit NSAID kaum zu behandeln, bzw. nur unter Inkaufnahme schwerer Nebenwirkungen beim Einsatz von Immunsuppressiva wie z.B. Cyclophosphamid, Cyclosporin A, Methotrexat oder Glukokortikoide. Diese können u.a. zu Nausea, Stomatitis, Veränderungen im Blutbild und Hepato- oder Nephrotoxizität führen.

Im U.S.-Patent 4 066 614 werden 3,5-Dialkyl-4-hydroxybenzoesäure-Derivate beschrieben, aber keine mit Homopiperazin substituierte Verbindungen. Im U.S.-Patent 4 128 664 werden 2,6-Di-(t-butyl)phenole, die in Position 4 des Phenolringes durch N-substituierte-Carboxyamid-Reste derivatisiert sind, beschrieben, die eine entzündungshemmende Wirkung haben.

Es wurde nun überraschend gefunden, daß 1-(4-Hydroxy-3,5-di-tert.-butylbenzoyl)-homopiperazin und verschiedene von dessen am Stickstoff substituierte Derivaten diese bisher kaum therapierbaren chronischen Graft-versus-Host-Krankheiten und die durch Immunkomplex-mediierten Autoimmunerkrankungen bei sehr guter Verträglichkeit, vorteilhafter therapeutischer Breite und erhaltener antiphlogistischer Eigenschaft günstig zu beeinflussen vermögen. Im Gegensatz hierzu weisen die aus der US-Patentschrift 4,128,664 bekannten 3,5-di-tert.-Butyl-4-hydroxybenzoesäureamide nur antiphlogistische Aktivität auf, eignen sich also nicht zur Behandlung der genannten schwerer Erkrankungen.

Gegenstand der vorliegenden Erfindung ist 1-(4-Hydroxy3,5-di-tert.-butylbenzoyl)-homopiperazin und dessen am Stickstoff substituierte Derivate der folgenden allgemeinen Formel I

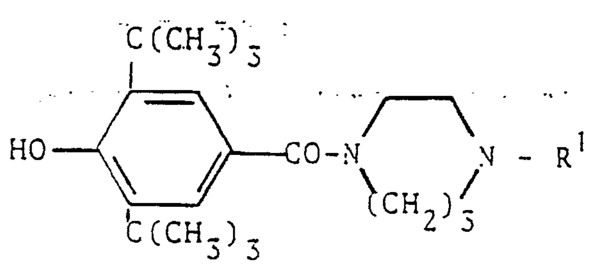

(I)

worin

$R^1$ = H,

einen geradkettigen oder verzweigten ($C_1$-$C_6$)-Alkoxycarbonylrest ($CH_3OCO$, $C_2$-$H_5OCO$, tert.-$C_4H_9OCO$, etc.)

oder den Benzyloxycarbonylrest, der im Phenylrest substituiert sein kann, darstellt,

sowie deren physiologisch verträglichen Salze.

Als Substituenten des Phenylkerns im Benzyloxycarbonylrest kommen in Frage:

Halogen, (F, Cl, Br, J, vorzugsweise Cl),

($C_1$-$C_4$)-Alkyl,

($C_1$-$C_4$)-Alkoxy,

($C_1$-$C_3$)Halogenalkyl, wie $CF_3$, $C_2H_4Cl$ etc.).

Von den Verbindungen der Formel I sind diejenigen bevorzugt, bei denen $R^1$ = H, ein geradkettiger

oder verzweigter ($C_1$-$C_4$)-Alkoxycarbonylrest oder der (im Phenylrest unsubstituierte) Benzyloxycarbonylrest ist; besonders bevorzugt ist $R^1$ nur = H oder der (im Phenylrest unsubstituierte) Benzyloxycarbonylrest.

Die Verbindungen der Formel I werden vorteilhaft hergestellt durch Umsetzung von 3,5-di-tert.-butyl-4-hydroxybenzoesäure und deren Derivaten der Formel II

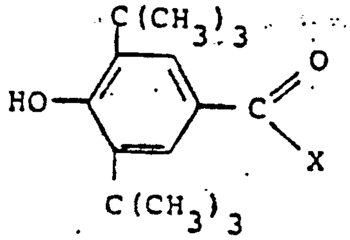

(II)

worin X = OH
Halogen (F, Cl, Br, J),
$N_3$,
OAlkyl (vorzugsweise $OC_1$-$C_4$-Alkyl),
OAryl (vorzugsweise $OC_6H_5$).

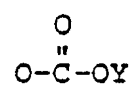

(mit Y = Alkyl, Aryl, Aralkyl, vorzugsweise $C_1$-$C_4$-Alkyl, $C_6H_5$, $CH_2C_6H_5$),

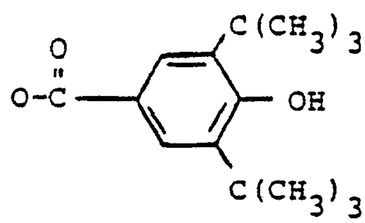

mit Aminen der Formel III

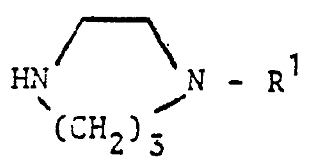

III

worin $R^1$ die gleiche Bedeutung wie in Formel I besitzt.

Besonders bevorzugte Ausgangsverbindung der Formel II ist das 3,5-Di-tert.-butyl-4-hydroxy-benzoesäurechlorid.

Die 3,5-Di-tert.-butyl-4-hydroxy-benzoesäure selbst kann in bekannter Weise, z.B. mittels der Kolbe'schen Synthese aus dem entsprechenden Phenol (2,6-Di-tert.-butylphenol) und $CO_2$, durch Cannizzaro-Reaktion oder auf oxidativem Weg, z.B. aus dem entsprechenden Aldehyd oder der Methylverbindung gewonnen werden. Aus der Säure sind ihre entsprechenden Derivate der Formel II nach üblichen Verfahren erhältlich. 2,6-Di-tert.-butylphenol ist ein am Markt erhältliches Produkt.

Als Ausgangsamine der Formel III kommen beispielsweise in Frage: Homopiperazin, N-Äthoxycarbonylhomopiperazin, N-tert.-Butyloxycarbonyl- und N-Benzyloxycarbonylhomopiperazin.

Im Fall des Einsatzes der Ausgangsverbindungen der Formel II mit X = OH, Halogen, $N_3$, OAlkyl, OAryl oder OC(O)OY werden diese Verbindungen und die Amine der Formel III vorzugsweise in einem Molverhältnis von 1: mindestens etwa 1 verwendet; im Falle des Einsatzes des 3,5-Di-tert.-butyl-benzoesäureanhydrids als Ausgangsverbindung der Formel II beträgt das Molverhältnis zu dem Amin III vorzugsweise 1:mindestens etwa 2.

Die Umsetzung kann ohne Lösungsmittel ausgeführt werden. Vorteilhaft arbeitet man jedoch unter Zusatz eines indifferenten Verteilungs- oder Lösungsmittels wie z.B. eines aliphatischen Säureamids (Dimethylformamid, Dimethylacetamid, etc.), Nitrils (Acetonitril, etc.), Ethers (Diethyl- oder Diisopropylether, Tetrahydrofuran, Dioxan, Glykoldiethylether, Diethylenglykoldimethylether, etc.) oder Alkohols (Methanol, Ethanol, Propanol, Isopropanol, etc.). Es kann ferner vorteilhaft sein, die Reaktion beispielsweise durch Basenzusatz oder durch katalytische Mengen von Dimethylformamid etc. zu beschleunigen. Wenn als Ausgangsverbindungen der Formel II die Säurehalogenide - insbesondere das Säurechlorid - verwendet werden, ist es vorteilhaft, die Umsetzung in Gegenwart von säurebindenden Mitteln, wie Alkali- oder Erdalkalicarbonaten, - hydroxiden oder -alkoholaten, organischen Basen (Triethylamin, Pyridin, Picolin, Chinolin, etc.) oder dem im Überschuß eingesetzten Amin der Formel III durchzuführen.

Die Reaktionstemperaturen liegen zweckmäßig zwischen etwa 0 und 160 °C, vorzugsweise zwischen etwa 20 und 80 °C.

Wenn die Amide der Formel I aus Estern hergestellt werden, kann sich die Verwendung von aktivierten Estern wie z.B. Cyanmethyl- oder Carboxymethylestern gegebenenfalls als günstig erweisen. Wird die freie Carbonsäure (Verbindung der Formel II mit X = OH) zu den erfindungsgemäßen Verbindungen der Formel I umgesetzt, ist die Verwendung wasserbindender Reagentien wie beispielsweise von Dicyclohexylcarbodiimid empfehlenswert.

Für die Herstellung der erfindungsgemäßen Verbindung der Formel I mit freier NH-Funktion (= 1-(4-Hydroxy-3,5-di-tert.-butylbenzoyl)-homopiperazin) kann es vorteilhaft sein, die NH-Gruppe mit einem leicht abspaltbaren Rest zu schützen, z.B. mit der Carbobenzoxygruppierung oder einer anderen - möglichst aktivierten - Ester- oder Acylgruppierung, und diese nach der Reaktion mit einer Verbindung der Formel II unter (hydrogenolytischer oder hydrolytischer) Freisetzung der NH-Funktion abzuspalten.

Insbesondere wenn die Verbindung der Formel I die freie basische NH-Funktion enthält, ist es vorteilhaft, das Endprodukt als Salz physiologisch verträglicher Säuren zu isolieren. Als Salze kommen für die therapeutische Anwendung beispielsweise das Hydrochlorid, das neutrale oder saure Sulfat, das primäre, sekundäre oder tertiäre Phosphat, das Methan- oder p-Toluolsulfonat oder auch das Salz einer organischen Säure wie beispielsweise das Maleat oder Zitrat in Frage. Besonders vorteilhaft ist die Verwendung des wasserlöslichen Hydrochlorids, das nach bekannten Verfahren - beispielsweise durch Umsetzung der basischen Verbindung I in alkoholischer Lösung oder Suspension mit der vorzugsweisen äquivalenten Menge alkoholischer oder wässriger Salzsäure-hergestellt wird.

Die erfindungsgemäßen Verbindungen der Formel I und die entsprechenden physiologisch verträglichen Säureadditionssalze eignen sich zur Anwendung als Heilmittel bzw. Wirkstoffe in Heilmitteln. Sie zeigen antiphlogistische Wirkungen, zeichnen sich aber vor allem dadurch aus, daß sie zur Behandlung von chronischen Graft-versus-Host-Krankheiten und von Immunkomplex-mediierten Autoimmunerkrankungen bei günstiger therapeutischer Breite geeignet sind. Sie sind ferner sehr gut verträglich.

Die entsprechenden Arzneimittel sind gekennzeichnet, durch einen Gehalt an - oder bestehen aus - mindestens einer Verbindung der Formel I und/oder mindestens einem ihrer physiologisch verträglichen Säureadditionssalze; sie werden hauptsächlich zur Vorbeugung und Behandlung von Erkrankungen des rheumatischen Formenkreises, bevorzugt bei den schweren Verlaufsformen verwendet.

Die Arzneimittel werden dadurch hergestellt, daß man mindestens eine Verbindung der Formel I und/oder mindestens ein physiologisch verträgliches Säureadditionssalz einer solchen Verbindung und/oder mindestens eine der nach dem vorstehend beschriebenen Verfahren erhaltenen Verbindungen mit einem physiologisch verträglichen Träger und gegebenenfalls weiteren Zusatz- und/oder Hilfsstoffen in eine geeignete Darreichungsform bringt.

Als Zubereitungsformen kommen vor allem Tabletten, Dragees, Kapseln und Suppositorien in Frage; sie können die Wirkstoffe entweder in freier Form (Verbindungen der Formel I) oder in Form der entsprechenden physiologisch verträglichen Säureadditionssalze enthalten. Für die intravenöse Applikation kommen vor allem wässrige Lösungen der Salze, die gegebenenfalls noch Lösungsvermittler enthalten können, zur Anwendung.

Alle diese Zubereitungen können noch andere therapeutisch aktive Komponenten, wie z. B. Analgetika, mit enthalten.

Die folgenden Beispiele sollen der weiteren Erläuterung der Erfindung dienen.

Beispiel 1:

1-(4-Hydroxy-3,5-di-tert.butyl-benzoyl)-4-(benzyl-oxycarbonyl)-homopiperazin

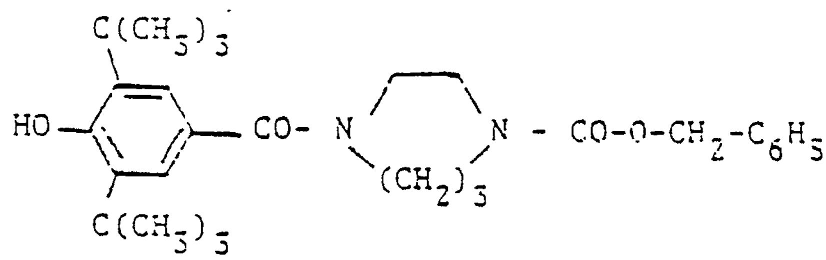

Eine Mischung von 13,4 g (0,05 mol) 3,5-Di-tert.butyl-4-hydroxybenzoesäurechlorid und 12,4 g (0,053 mol) 1-Benzyl-oxycarbonyl-homopiperazin in 200 ml Acetonitril wird unter Rühren 6 Stunden am Rückfluß erhitzt. Anschließend wird das Lösungsmittel unter verminderten Druck abdestilliert, der Rückstand zwischen Methylenchlorid und verdünnter Salzsäure verteilt, abgetrennt und die organische Phase mit gesättigter Kochsalzlösung gewaschen. Nach dem Trocknen über Natriumsulfat und dem Abdestillieren des Lösungsmittels unter vermindertem Druck wird der kristalline Rückstand mit heißen Diisopropyläther digeriert, abgesaugt, mit Diisopropyläther gewaschen und getrocknet.
Ausbeute: 16 g (69 % d. Th.)
Schmelzpunkt: 146 °C
$C_{28}H_{38}N_2O_4$ (MG = 466,6)

Analyse:

Berechnet: C 72,07 % H 8,21 % N 6,00 %
Gefunden: C 71,76 % H 8,31 % N 5,97 %

Beispiel 2:

1-(4-Hydroxy-3,5-di-tert.butyl-benzoyl)-homopiperazin

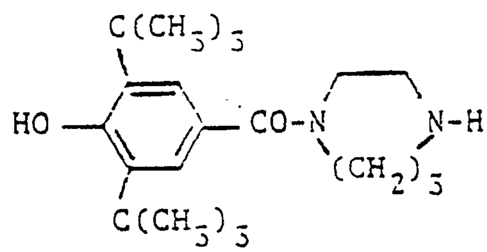

Eine Mischung von 46,6 g (0,1 mol) 1-(4-Hydroxy-3,5-di-tert.butyl-benzoyl)-4-(benzyl-oxycarbonyl)-homopiperazin (Beispiel 1),350 ml Eisessig und 2,3 g Palladiummohr werden unter Rühren bei 70-80 °C etwa 7 Stunden lang mit Wasserstoff behandelt. Anschließend wird vom Katalysator filtriert und das Lösungsmittel unter vermindertem Druck abdestilliert. Der Rückstand wird in Methylenchlorid gelöst, mit verdünnter Natronlauge ausgeschüttelt und abgetrennt. Die organische Phase wird mehrmals mit Wasser gewaschen und sodann über wasserfreiem Natriumsulfat getrocknet. Der nach Abziehen des Lösungsmittels verbleibende Rückstand wird aus Acetonitril unter Zusatz von Aktivkohle umkristallisiert.
Ausbeute: 25 g (75 % d. Th.)
Schmelzpunkt: 124 °C
$C_{20}H_{32}N_2O_2$ (MG = 332,5)

Analyse:

Berechnet: C 72,25 % H 9,7 % N 8,43 %
Gefunden: C 72,10 % H 9,58 % N 8,52 %

Pharmakologische Prüfung und Ergebnisse

Die Prüfung der erfindungsgemäßen Verbindungen der Formel I auf antiphlogistische Wirkung wurde in der Adjuvans-Arthritis, die auf Beeinflussung der schweren Verlaufsformen von Erkrankungen des rheumatischen Formenkreises (chronische Graft-versus-Host-Krankheiten und Immunkomplexmediierte Autoimmunerkrankungen) wurden in der chronischen Graft-versus-Host (cGvH)-Reaktion an der Maus bzw. in der Reverse Passive Arthus-Reaktion an der Ratte entsprechend den unten beschriebenen Tiermodellen durchgeführt. Die Bestimmung der Verträglichkeit erfolgte durch Toxizitätsbestimmung an der Ratte, und zwar durch kontinuierliche Gabe über 14 Tage.

1. Adjuvans-Arthritis

Die Untersuchungen wurden nach der Methode von Pearson (Arthrit. Rheum. 2 (1959) S. 44) durchgeführt. Als Versuchstiere dienten männliche Ratten eines Wistar-Lewis-Stammes mit einem Körpergewicht zwischen 130 und 200 g. Die zu prüfenden Verbindungen wurden in Dosen von 50 mg pro kg Körpergewicht einmal täglich vom 1. bis zum 3. Versuchstag oral (p. o.) appliziert. Die Tiere einer Kontrollgruppe erhielten nur das Vehikel. Jede Präparat- und Kontrollgruppe umfaßte 8 Tiere. Als Wirkungskriterium diente die prozentuale Herabsetzung der Pfotenvolumenzunahme gegenüber jener der unbehandelten Kontrollgruppe.

| Verbindung aus Beispiel | Dosis (mg / kg p.o.) | % Hemmung Pfotenvolumen 5.Tag |
|---|---|---|
| 1 | 50 | 25 |
| 2 | 50 | 34 |

Die Substanzen bewirken eine signifikante Hemmung der Adjuvans-induzierten Arthritis an der Ratte.

2. Chronische Graft-versus-Host(cGvH)-Reaktion an der Maus

Die Graft-versus-Host-Krankheit, der eine vom Transplantat ausgehende, gegen das Wirtsgewebe gerichtete Immunreaktion zugrunde liegt, ist bei akuter, fast immer tödlich endender Verlaufsform durch Milzvergrößerung, Leberschwellung, Hypertrophie der Lymphknoten, hämolytische Anämie, erniedrigte Immunglobulin- und Komplementspiegel sowie verminderte Immunreaktivität gekennzeichnet. Die etwas milder verlaufende chronische Krankheitsform führt zu Lymphadenopathie, Immunkomplexglomerulonephritis und zur exzessiven Bildung von organunspezifischen Autoantikörpern. Ein ähnliches Krankheitsbild kennzeichnet den systemischen Lupus erythematodes (SLE), der ebenfalls zum Formenkreis der Autoimmunerkrankungen gehört.

Die Untersuchung der erfindungsgemäß verwendeten Verbindungen auf den Krankheitsverlauf der durch zwei Injektionen von miteinander gemischten Milz- und Thymuszellen an weiblichen Mäusen der (DBA/2 x C57Bl/6)Fl-Generation ausgelösten cGvH-Reaktion erfolgte in der von S.Popovic und R.R. Bartlett beschriebenen Versuchsanordnung (Agents and Actions 21 (1987), 284- 286), wobei im zeitlichen Abstand von 7 Tagen jeweils 5 x $10^7$ ebenfalls von weiblichen Spendertieren gewonnene DBA/2-Zellen in 0,2 ml Kulturmedium intravenös verabreicht wurden. Zur sicheren Beurteilung des Krankheitsverlaufes und -ausbruches wurde bei allen Versuchen ein Kollektiv gesunder Tiere als Negativkontrolle mitgeführt. Die 6-wöchige orale Behandlung der kranken Tiere erfolgte ab dem 21. Tage nach der ersten. Donorzellen-Injektion, wobei die Prüfsubstanz oder das reine Vehikel (Positivkontrolle) einmal täglich appliziert wurden. Als Vehikel diente eine wäßrige CMC(Carboxymethylcellulose-Natriumsalz)-Lösung mit einem Gehalt von 100 mg CMC pro l. Das Applikationsvolumen betrug 10 ml pro kg-Körpergewicht. Die einzelnen Versuchskollektive umfaßten jeweils 10 Tiere.

Die Präparatewirkung wurde anhand der Hemmung der Proteinurie und des cGvH-Index beurteilt. Die kranken Tiere entwickeln infolge der Zerstörung von Nephronen durch Immunkomplexablagerung an der Basalmembrane der Glomeruli eine ausgeprägte Proteinurie, die mit dem Ausmaß der Glomerulonephritis

korreliert und die sich über den Anstieg der mit dem Urin ausgeschiedenen Proteinmenge leicht quantifizieren läßt. Der zweite Meßparameter, der cGvH-Index, bezieht sich auf die durch die cGvH-Reaktion hervorgerufene starke Milzvergrößerung (Splenomegalie). Er ist definiert als der Quotient aus dem Produkt von Milz- und Körpergewicht der kranken Tiere und dem Produkt der entsprechenden Gewichte gesunder, unbehandelter Tiere aus der Negativkontrollgruppe und stellt ein verläßliches Maß für die Krankheitsintensität dar (je größer dieser Index, desto größer die Krankheit).

Die in der Tabelle zusammengefaßten Ergebnisse dieser Untersuchungen belegen, daß Verbindungen der Formel I die cGvH-Krankheit durch modulierenden Eingriff in die Autoimmunprozesse nachhaltig zu lindern vermögen. NSAID zeigen in diesem Test keine Wirkung.

| Verbindung aus Beispiel | Dosis in mg/kg/Tag p.o. | % Hemmung | |
|---|---|---|---|
| | | Proteinurie | cGvH-Index |
| 1 | 5 | 100 | 57 |
| | 20 | 80 | 75 |

3. Reverse Passive Arthus-Reaktion an der Ratte

Als Versuchstiere dienten weibliche und männliche Sprague-Dawley-Ratten mit einem Körpergewicht zwischen 80 und 100 g. Die Ratten wurden in Gruppen von jeweils 8 Tieren aufgeteilt. 1 Stunde vor Auslösung der Arthus-Reaktion durch Injektion von 0,1 ml einer Anti-Ratten-IgG-Lösung (0,6 mg/ml) in die rechte Hinterpfote erfolgte die orale Verabreichung der jeweiligen Prüfsubstanz oder des reinen Vehikels (Positivkontrolle). In die linke Pfote wurde Natriumchlorid-Lösung injiziert. Eine Gruppe nicht sensibilisierter Tiere (Negativkontrolle) wurde gleichfalls mit Ovalbumin behandelt, um unspezifische Reaktionen gegenüber dem Protein ausschließen zu können. Als Meßparameter für die Präparatewirkung diente die prozentuale Veränderung der Pfotenvolumenzunahme gegenüber jener der zwar sensibilisierten, aber unbehandelten Kontrollgruppe (Positivkontrolle) 4 Stunden nach der Ovalbumin-Provokation, wenn die Schwellung ihren Maximalwert erreicht.

| Verbindung aus Beispiel | Reverse Passive Arthus-Reaktion | |
|---|---|---|
| | Dosis in mg/kg p.o. | % Hemmung |
| 1 | 100 | 25 |
| 2 | 20 | 28 |
| | 25 | 37 |

4. Toxizitätsbestimmungen an der Ratte

Je 10 männliche und weibliche Wistarratten wurden für die Dauer von 14 Tagen täglich mit je 50 und 75 mg/kg p.o. gefüttert und alle 2 Tage gewogen. Nach 14 Tagen wurde der Versuch abgebrochen und die Gewichtszunahme bestimmt. Als Kontrolle dienten die gleiche Anzahl nicht Präparate-be handelter Ratten. Die Verbindungen der Boispiele 1 und 2 führten zu keiner signifikanten Anderung der Gewichte. Dieses wird als kritischer Parameter für die Verträglichkeit einer Verbindung angesehen.

**Patentansprüche**

**Patentansprüche für folgende Vertragsstaaten : AT, BE, CH, DE, FR, GB, IT, LI, LU, NL, SE**

**1.** 1-(4-Hydroxy-3,5-di-tert.-butyl-benzoyl)-homopiperazin und dessen am N substituierte Derivate der allgemeinen Formel I

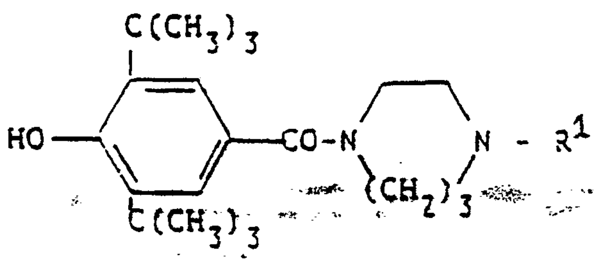

(I)

in der
$R^1$ ein Wasserstoffatom oder einen geradkettigen oder verzweigten $(C_1-C_6)$-Alkyloxycarbonylrest oder den Benzyloxycarbonylrest, der im Phenylkern durch $(C_1-C_4)$-Alkyl, $(C_1-C_4)$-Alkoxy, Halogen oder $(C_1-C_3)$-Halogenalkyl, wobei Halogen für Fluor, Chlor, Brom oder Jod steht, substituiert sein kann, darstellt sowie deren physiologisch verträglichen Säureadditionssalze.

**2.** Verbindungen und deren Säureadditionssalze nach Anspruch 1, dadurch gekennzeichnet, daß in Formel I der Rest $R^1$ ein Wasserstoffatom oder einen geradkettigen oder verzweigten $(C_1-C_6)$-Alkyloxycarbonylrest oder den Benzyloxycarbonylrest bedeutet.

**3.** Verbindungen und deren Säureadditionssalze nach Anspruch 1, dadurch gekennzeichnet, daß in Formel I der Rest $R^1$ ein Wasserstoffatom oder den Benzyloxycarbonylrest darstellt.

**4.** Verfahren zur Herstellung von 1-(4-Hydroxy-3,5-di-tert.-butylbenzoyl)-homopiperazin und dessen am Stickstoff substituierten Derivaten der Formel I sowie der physiologisch verträglichen Säureadditionssalze dieser Verbindungen gemäß einem oder mehreren der Ansprüche 1 - 3, dadurch gekennzeichnet, daß man

a) 3,5-Di-tert.-butyl-4-hydroxy-benzoesäure(derivate) der Formel II

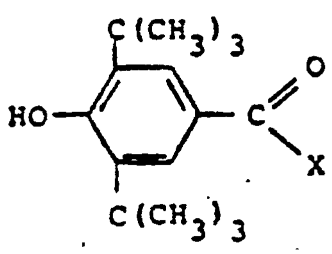

(II)

worin X = OH,
Halogen (F, Cl, Br, J),
$N_3$,
OAlkyl,
OAryl,

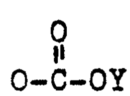

(mit Y = Alkyl, Aryl, Aralkyl), oder

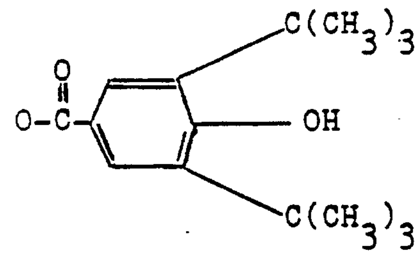

mit Aminen der Formel III

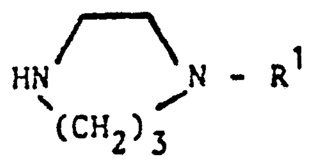 (III)

worin $R^1$ die gleiche Bedeutung wie in Formel I besitzt, zu den Verbindungen der Formel I umsetzt oder

b) zur Herstellung der Verbindung der Formel I mit $R^1$ = H von der Verbindung der Formel I mit $R^1$ = Benzyloxycarbonyl oder ein anderer leicht abspaltbarer Rest, ausgeht und den Benzyloxycarbonyl- bzw. den anderen leicht abspaltbaren Rest auf bekannte Weise hydrogenolytisch oder hydrolytisch abspaltet,

und die Verbindung der Formel I gegebenenfalls mit geeigneten Säuren in ihre physiologisch verträglichen Säureadditionssalze überführt.

**5.** Verfahren nach Anspruch 4, dadurch gekennzeichnet, daß man von Verbindungen der Formel II mit X = Cl ausgeht und die Umsetzung in Gegenwart eines säurebindenden Mittels durchführt.

**6.** Verbindungen der Formel I sowie deren physiologisch verträgliche Säureadditionssalze gemäß einem oder mehreren der Ansprüche 1 - 3 oder wie erhalten nach Anspruch 4 und/oder 5 zur Anwendung als Heilmittel.

**7.** Arzneimittel, gekennzeichnet durch einen Gehalt an - oder bestehend aus - mindestens einer Verbindung der Formel I und/oder mindestens einem von deren physiologisch verträglichen Säureadditionssalzen nach einem oder mehreren der Ansprüche 1 - 3 und/oder mindestens einer nach dem Verfahren gemäß Anspruch 4 oder 5 hergestellten Verbindung.

**8.** Arzneimittel nach Anspruch 7, dadurch gekennzeichnet, daß es zur Vorbeugung und Behandlung von entzündlichen Erkrankungen, vorzugsweise des rheumatischen Formenkreises, verwendet wird.

**9.** Verfahren zur Herstellung eines Arzneimittels gemäß einem der - oder beiden - Ansprüche (n) 7 und 8, dadurch gekennzeichnet, daß man mindestens eine Verbindung der Formel I und/oder mindestens ein physiologisch verträgliches Säureadditionssalz einer solchen Verbindung und/oder mindestens eine der nach dem Verfahren gemäß Anspruch 4 und/oder 5 erhaltenen Verbindung mit einem physiologisch verträglichen Träger und gegebenenfalls weiteren Zusatz- und/oder Hilfsstoffen in eine geeignete Darreichungsform bringt.

**10.** Verwendung von Verbindungen der Formel I und/oder deren physiologisch verträglichen Säureadditionssalzen zur Herstellung von Arzneimitteln gemäß den Ansprüche 7 und 8.

**Patentansprüche für folgende Vertragsstaaten : ES, GR**

**1.** Verfahren zur Herstellung von 1-(4-Hydroxy-3,5-di-tert.-butyl-benzoyl)-homopiperazin und dessen am Stickstoff substituierten Derivaten der allgemeinen Formel I

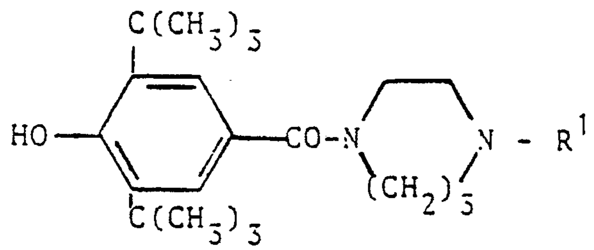

(I)

in der
$R^1$ ein Wasserstoffatom oder einen geradkettigen oder verzweigten ($C_1$-$C_6$)-Alkoxycarbonylrest oder den Benzyloxycarbonylrest, der im Phenylkern durch ($C_1$-$C_4$)-Alkyl, ($C_1$-$C_4$)Alkoxy, Halogen oder ($C_1$-$C_3$)-Halogenalkyl, wobei Halogen für Fluor, Chlor, Brom oder Jod steht, substituiert sein kann, darstellt sowie der physiologisch verträglichen Säureadditionssalze dieser Verbindungen,
dadurch gekennzeichnet,

a) 3,5-Di-tert.-butyl-4-hydroxy-benzoesäure(derivate) der Formel II

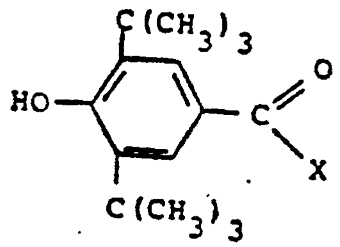

(II)

worin X = OH,
Halogen, (F, Cl, Br, J),
$N_3$
OAlkyl,
OAryl.

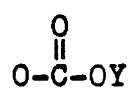

(mit Y = Alkyl, Aryl, Aralkyl), oder

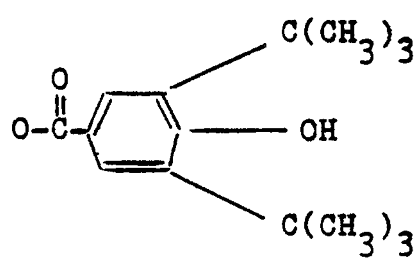

mit Aminen der Formel III

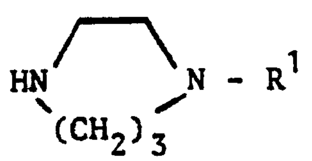

(III)

worin $R^1$ die gleiche Bedeutung wie in Formel I besitzt, zu den Verbindungen der Formel I umsetzt

oder
b) zur Herstellung der Verbindung der Formel I mit $R^1$ = H von der Verbindung der Formel I mit $R^1$ = Benzyloxycarbonyl oder ein anderer leicht abspaltbarer Rest, ausgeht und den Benzyloxycarbonyl- bzw. den anderen leicht abspaltbaren Rest auf bekannte Weise hydrogenolytisch oder hydrolytisch abspaltet.

2. Verfahren nach Anspruch 1, dadurch gekennzeichnet, daß man Verbindungen der Formel I mit $R^1$ = H oder geradkettiger oder verzweigeter ($C_1$-$C_6$)-Alkoxycarbonylrest oder Benzyloxycarbonylrest, herstellt.

3. Verfahren nach Anspruch 1 oder 2, dadurch gekennzeichnet, daß man Verbindungen der Formel I mit $R^1$ = H oder Benzyloxycarbonylrest, herstellt.

4. Verfahren nach einem oder mehreren der Ansprüche 1 - 3, dadurch gekennzeichnet, daß man von Verbindungen der Formel II mit X = Cl ausgeht und die Umsetzung in Gegenwart eines säurebindenden Mittels durchführt.

5. Verfahren zur Herstellung der Verbindungen der Formel I sowie deren physionogisch verträglicher Säureadditionssalze zur Anwendung als Heilmittel.

6. Verfahren zur Herstellung eines Arzneimittels, dadurch gekennzeichnet, daß man mindestens eine Verbindung der Formel I gemäß der Definition in Anspruch 1 und/oder mindestens ein physiologisch verträgliches Säureadditionssalz einer solchen Verbindung und/oder mindestens eine der nach dem Verfahren gemäß einem oder mehreren der Ansprüche 1 - 4 erhaltenen Verbindungen mit einem physiologisch verträglichen Träger und gegebenenfalls weiteren Zusatz- und/oder Hilfsstoffen in eine geeignete Darreichungsform bringt.

7. Verfahren nach Anspruch 6, dadurch gekennzeichnet, daß das verfahrensgemäß hergestellte Arzneimittel zur Vorbeugung und Behandlung von entzündlichen - insbesondere von entzündlich rheumatischen - Erkrankungen bestimmt ist.

8. Verwendung von Verbindungen der Formel I gemäß der Definition in Anspruch 1 oder wie erhalten nach einem oder mehreren der Ansprüche 1 - 4, und/oder der physiologisch verträglichen Säureadditionssalze dieser Verbindungen zur Herstellung von Arzneimitteln zur Vorbeugung und Behandlung der in Anspruch 7 genannten Erkrankungen.

## Claims

### Claims for the following Contracting States : AT, BE, CH, DE, FR, GB, IT, LI, LU, NL, SE

1. 1-(4-Hydroxy-3,5-di-tert.-butylbenzoyl)homopiperazine and derivatives thereof substituted on the nitrogen, of the formula I

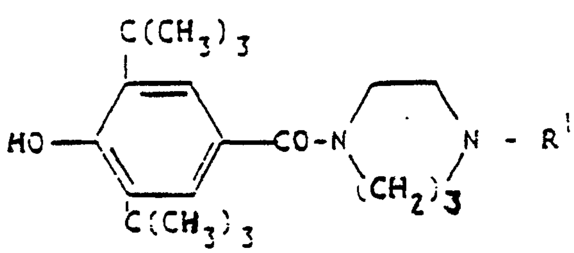

(I)

in which
$R^1$ represents a hydrogen atom or a straight-chain or branched ($C_1$-$C_6$)-alkyloxycarbonyl radical, or the benzyloxycarbonyl radical, which can be substituted in the phenyl nucleus by ($C_1$-$C_4$)-alkyl, ($C_1$-$C_4$)-alkoxy, halogen or ($C_1$-$C_3$)-halogenoalkyl, where halogen represents fluorine, chlorine, bromine or iodine,
and their physiologically tolerated acid addition salts.

2. A compound and its acid addition salts as claimed in claim 1, wherein in formula I the radical $R^1$ denotes a hydrogen atom or a straight-chain or branched ($C_1$-$C_6$)-alkyloxycarbonyl radical or the benzyloxycarbonyl radical.

3. A compound and its acid addition salts as claimed in claim 1, wherein in formula I the radical $R^1$ represents a hydrogen atom or the benzyloxycarbonyl radical.

4. A process for the preparation of 1-(4-hydroxy-3,5-di-tert.-butylbenzoyl)homopiperazine and derivatives thereof substituted on the nitrogen, of the formula I, and of the physiologically tolerated acid addition salts of these compounds as claimed in one or more of claims 1 - 3, which comprises
a) reaction of 3,5-di-tert.-butyl-4-hydroxybenzoic acid (derivatives) of the formula II

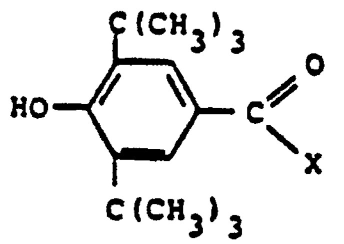

(II)

in which X = OH,
halogen (F, Cl, Br or I),
$N_3$,
Oalkyl,
Oaryl,

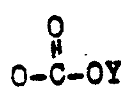

(with Y = alkyl, aryl, aralkyl), or

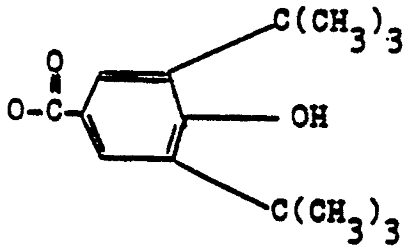

with amines of the formula III

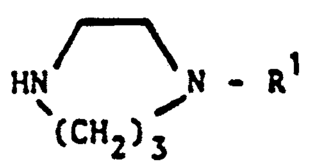

(III)

in which $R^1$ has the same meaning as in formula I, to give the compounds of the formula I, or
b) for the preparation of the compound of the formula I with $R^1$ = H, starting the process from the compound of the formula I with $R^1$ = benzyloxycarbonyl or another radical which can readily be eliminated, and elimination, in a known manner by hydrogenolysis or hydrolysis, of the benzyloxycarbonyl or the other radical which can readily be eliminated, and conversion, where appropriate, of the compound of the formula I with suitable acids into its physiologically tolerated acid addition salts.

5. The process as claimed in claim 4, which starts from compounds of the formula II with X = Cl, and in which the reaction is carried out in the presence of an acid-binding agent.

6. A compound of the formula I and its physiologically tolerated acid addition salts as claimed in one or more of claims 1 - 3, or as obtained as claimed in claim 4 and/or 5, for use as medicines.

7. A medicament containing - or comprising - at least one compound of the formula I and/or at least one of its physiologically tolerated acid addition salts as claimed in one or more of claims 1 - 3, and/or at least one compound prepared by the process as claimed in claim 4 or 5.

8. A medicament as claimed in claim 7, which is used for the prevention and treatment of inflammatory disorders, preferably rheumatic disorders.

9. A process for the preparation of a medicament as claimed in one - or both - of claims 7 and 8, which comprises converting at least one compound of the formula I, and/or at least one physiologically tolerated acid addition salt of such a compound, and/or at least one of the compounds obtained by the process as claimed in claim 4 and/or 5, into a suitable dosage form with a physiologically tolerated vehicle and, where appropriate, further additives and/or auxiliaries.

10. The use of compounds of the formula I and/or of their physiologically tolerated acid addition salts for the preparation of medicaments as claimed in claims 7 and 8.

**Claims for the following Contracting States : ES, GR**

1. A process for the preparation of 1-(4-Hydroxy-3,5-di-tert.-butylbenzoyl)homopiperazine and derivatives thereof substituted on the nitrogen, of the formula I

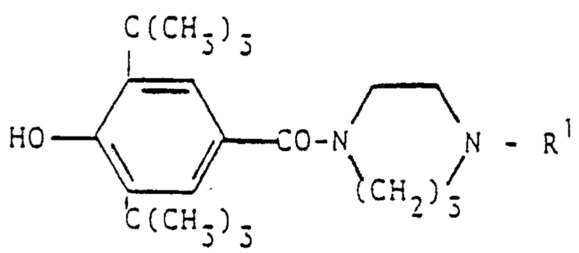

(I)

in which

$R^1$ represents a hydrogen atom or a straight-chain or branched ($C_1$-$C_6$)-alkoxycarbonyl radical, or the benzyloxycarbonyl radical, which can be substituted in the phenyl nucleus by ($C_1$-$C_4$)-alkyl, ($C_1$-$C_4$)-alkoxy, halogen or ($C_1$-$C_3$)-halogenoalkyl, where halogen represents fluorine, chlorine, bromine or iodine,

and the physiologically tolerated acid addition salts of these compounds,

which comprises

a) reaction of 3,5-di-tert.-butyl-4-hydroxybenzoic acid (derivatives) of the formula II

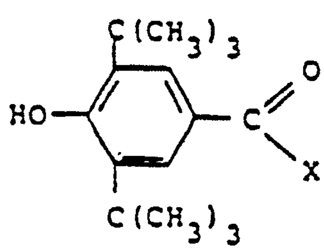

(II)

in which X = OH,
halogen (F, Cl, Br or I),
$N_3$,

Oalkyl,
Oaryl,

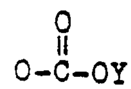

(with Y = alkyl, aryl, aralkyl),or

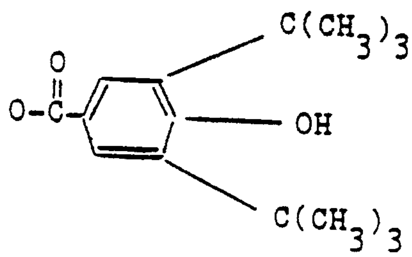

with amines of the formula III

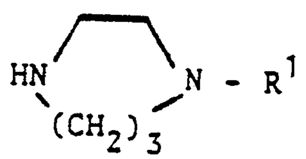

(III)

in which $R^1$ has the same meaning as in formula I, to give the compounds of the formula I, or
b) for the preparation of the compound of the formula I with $R^1$ = H, starting the process from the compound of the formula I with $R^1$ = benzyloxycarbonyl or another radical which can readily be eliminated, and elimination, in a known manner by hydrogenolysis or hydrolysis, of the benzyloxycarbonyl or the other radical which can readily be eliminated.

**2.** The process as claimed in claim 1, wherein compounds of the formula I with $R^1$ = H or straight-chain or branched ($C_1$-$C_6$)-alkoxycarbonyl radical or benzyloxycarbonyl radical are prepared.

**3.** The process as claimed in claim 1 or 2, wherein compounds of the formula I with $R^1$ = H or benzyloxycarbonyl radical are prepared.

**4.** The process as claimed in one or more of claims 1 - 3, which starts from compounds of the formula II with X = Cl, and in which the reaction is carried out in the presence of an acid-binding agent.

**5.** A process for the preparation of compounds of the formula I and the physiologically tolerated acid addition salts thereof for use as medicines.

**6.** A process for the preparation of a medicament which comprises converting at least one compound of the formula I, as defined in claim 1 and/or at least one physiologically tolerated acid addition salt of such a compound and/or at least one of the compounds obtained by the process as claimed in one or more of claims 1 - 4 into a suitable dosage form with a physiologically tolerated vehicle and, where appropriate, further additives and/or auxiliaries.

**7.** The process as claimed in claim 6, wherein the medicament prepared by the process is intended for the prevention and treatment of inflammatory - especially of inflammatory rheumatic - disorders.

**8.** The use of compounds of the formula I as defined in claim 1 or as obtained as claimed in one or more of claims 1 - 4, and/or of the physiologically tolerated acid addition salts of these compounds for the

preparation of medicaments for the prevention and treatment of the disorders mentioned in claim 7.

**Revendications**

**Revendications pour les Etats contractants suivants : AT, BE, CH, DE, FR, GB, IT, LI, LU, NL, SE**

1. 1-(4-hydroxy-3,5-di-tert-butylbenzoyl)-homo-pipérazine et ses dérivés substitués à l'azote, de formule générale I

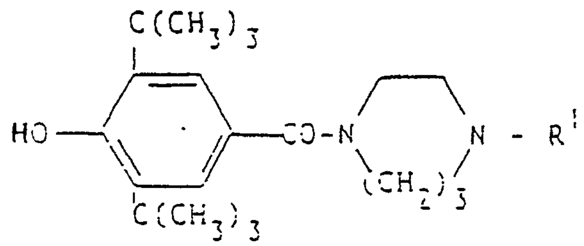

(I)

dans laquelle $R^1$ représente un atome d'hydrogène ou un radical alcoxy($C_1$-$C_6$)-carbonyle à chaîne droite ou ramifiée ou le radical benzyloxycarbonyle qui peut être substitué sur le noyau phényle par un atome d'halogène ou un groupe alkyle en $C_1$-$C_4$, alcoxy en $C_1$-$C_4$ ou halogénoalkyle en $C_1$-$C_3$, l'halogène représentant le fluor, le chlore, le brome ou l'iode,
ainsi que leurs sels d'addition avec des acides physiologiquement acceptables.

2. Composés et leurs sels d'addition avec des acides selon la revendication 1, caractérisés en ce que, dans la formule I, le radical $R^1$ représente un atome d'hydrogène ou un radical alcoxy($C_1$-$C_6$)-carbonyle à chaîne droite ou ramifiée ou le radical benzyloxycarbonyle.

3. Composés et leurs sels d'addition avec des acides selon la revendication 1, caractérisés en ce que dans la formule I, le radical $R^1$ représente un atome d'hydrogène ou le radical benzyloxycarbonyle.

4. Procédé pour la préparation de la 1-(4-hydroxy-3,5-di-tert-butylbenzoyl)-homopipérazine et de ses dérivés substitués à l'azote, de formule I, ainsi que des sels d'addition avec des acides physiologiquement acceptables de ces composés, selon une ou plusieurs des revendications 1 à 3, caractérisé en ce que
   a) on fait réagir des dérivés de l'acide 3,5-di-tert-butyl-4-hydroxybenzoïque de formule II

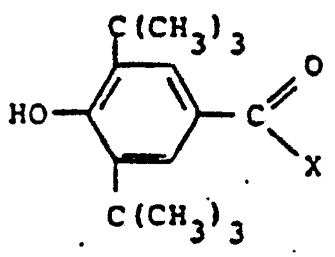

(II)

dans laquelle
X = OH,
un atome d'halogène (F, Cl, Br, I),
$N_3$,
un groupe O-alkyle,
un groupe O-aryle,

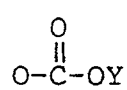

(avec Y = alkyle, aryle, aralkyle,), ou

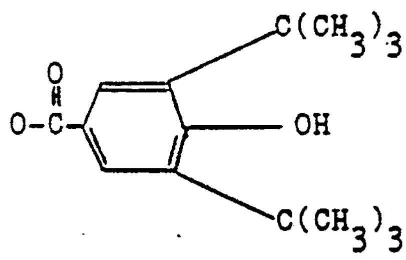

avec des amines de formule III

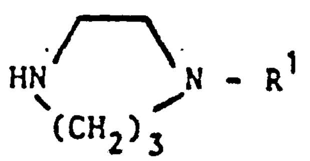

(III)

dans laquelle $R^1$ a la même signification que dans la formule I, pour aboutir aux composés de formule I, ou

b) pour la préparation du composé de formule I dans lequel $R^1$ = H, on part du composé de formule I dans lequel $R^1$ représente le radical benzyloxycarbonyle ou un autre radical aisément séparable, et on élimine par hydrolyse ou hydrogénolyse, de façon connue, le radical benzyloxycarbonyle ou l'autre radical aisément séparable,

et éventuellement on convertit le composé de formule I, à l'aide d'acides appropriés, en ses sels physiologiquement acceptables.

**5.** Procédé selon la revendication 4, caractérisé en ce que l'on part de composés de formule II dans lesquels X = Cl, et on effectue la réaction en présence d'un agent capteur d'acide.

**6.** Composés de formule I ainsi que leurs sels d'addition avec des acides physiologiquement acceptables, selon une ou plusieurs des revendications 1 à 3 ou tels qu'obtenus selon la revendication 4 et/ou la revendication 5, pour utilisation en tant que médicament.

**7.** Médicament caractérisé par une teneur en - ou consistant en - au moins un composé de formule I et/ou au moins un de ses sels d'addition avec des acides physiologiquement acceptables, selon une ou plusieurs des revendications 1 à 3 et/ou au moins un composé préparé par le procédé selon la revendication 4 ou 5.

**8.** Médicament selon la revendication 7, caractérisé en ce qu'il est utilisé pour la prévention et le traitement de maladies inflammatoires, de préférence du syndrome rhumatismal.

**9.** Procédé pour la fabrication d'un medicament selon l'une des revendications 7 et 8 ou les deux, caractérisé en ce que l'on met sous une forme pharmaceutique appropriée au moins un composé de formule I et/ou au moins un sel d'addition avec un acide physiologiquement acceptable d'un tel composé et/ou au moins un composé obtenu par le procédé selon la revendication 4 et/ou la revendication 5, avec un véhicule physiologiquement acceptable et éventuellement d'autres additifs et/ou adjuvants.

**10.** Utilisation des composés de formule I et/ou de leurs sels d'addition avec des acides physiologiquement acceptables, pour la fabrication de médicaments selon les revendications 7 et 8.

**Revendications pour les Etats contractants suivants : ES, GR**

**1.** Procédé pour la préparation de la 1-(4-hydroxy-3,5-di-tert-butylbenzoyl)-homopipérazine et de ses

dérivés substitués à l'azote, de formule générale I

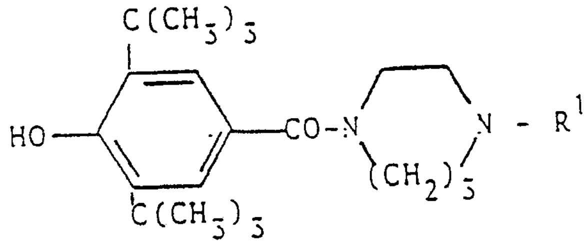

(I)

dans laquelle $R^1$ représente un atome d'hydrogène ou un radical alcoxy($C_1$-$C_6$)-carbonyle à chaîne droite ou ramifiée ou le radical benzyloxycarbonyle qui peut être substitué sur le noyau phényle par un atome d'Halogène ou un groupe alkyle en $C_1$-$C_4$, alcoxy en $C_1$-$C_4$ ou halogénoalkyle en $C_1$-$C_3$, l'halogène représentant le fluor, le chlore, le brome ou l'iode,
ainsi que de ses sels d'addition avec des acides physiologiquement acceptables,
caractérisé en ce que

a) on fait réagir des dérivés d'acide 3,5-di-tert-butyl-4-hydroxybenzoïque de formule II

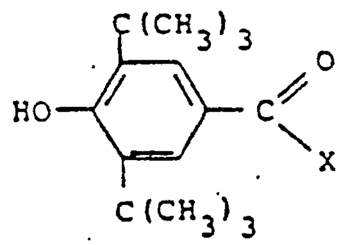

(II)

dans laquelle

X = OH,
un atome d'halogène (F, Cl, Br, I),
$N_3$,
un groupe O-alkyle,
un groupe O-aryle,

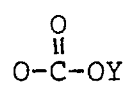

(avec Y = alkyle, aryle, aralkyle,), ou

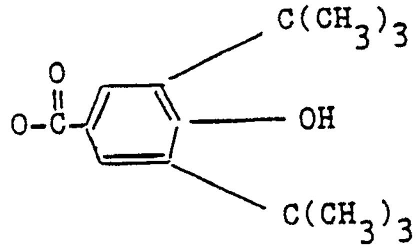

avec des amines de formule III

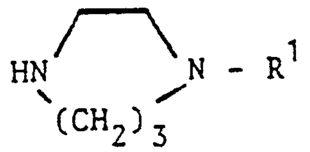 (III)

dans laquelle $R^1$ a la même signification que dans la formule I, pour aboutir aux composés de formule I, ou

b) pour la préparation du composé de formule I dans lequel $R^1$ = H, on part du composé de formule I dans lequel $R^1$ représente le radical benzyloxycarbonyle ou un autre radical aisément séparable, et on élimine par hydrolyse ou hydrogénolyse, de façon connue, le radical benzyloxycarbonyle ou l'autre radical aisément séparable.

**2.** Procédé selon la revendication 1, caractérisé en ce que l'on prépare des composés de formule I dans lesquels $R^1$ est un atome d'hydrogène ou un radical alcoxy($C_1$-$C_6$)-carbonyle à chaîne droite ou ramifiée ou benzyloxycarbonyle.

**3.** Procédé selon la revendication 1 ou 2, caractérisé en ce que l'on prépare des composés de formule I dans lesquels $R^1$ représente un atome d'hydrogène ou le radical benzyloxycarbonyle.

**4.** Procédé selon une ou plusieurs des revendications 1 à 3, caractérisé en ce que l'on part de composés de formule II dans lesquels X = Cl, et on effectue la réaction en présence d'un agent capteur d'acide.

**5.** Procédé pour la préparation des composés de formule I ainsi que de leurs sels d'addition avec des acides physiologiquement acceptables, pour utilisation en tant que médicament.

**6.** Procédé pour la fabrication d'un médicament, caractérisé en ce que l'on met sous une forme pharmaceutique appropriée au moins un composé de formule I selon la définition donnée dans la revendication 1 et/ou au moins un sel d'addition avec un acide physiologiquement acceptable d'un tel composé et/ou au moins un des composés obtenus par le procédé selon une ou plusieurs des revendications 1 à 4, avec un véhicule physiologiquement acceptable et éventuellement d'autres additifs et/ou adjuvants.

**7.** Procédé selon la revendication 6, caractérisé en ce que le médicament fabriqué selon le procédé est indiqué pour la prévention et le traitement de maladies inflammatoires - en particulier de maladies rhumatismales inflammatoires.

**8.** Utilisation des composés de formule I selon la définition donnée dans la revendication 1 ou tels qu'obtenus selon une ou plusieurs des revendicaitons 1 à 4 et/ou des sels d'addition avec des acides physiologiquement acceptables de ces composés, pour la fabrication de médicaments pour la prévention et le traitement des maladies mentionnées dans la revendication 7.